# FASCICULE DE BREVET EUROPEEN

(11) **EP 1 281 699 B1**
(45) Date de publication et mention de la délivrance du brevet: **01.12.2004**
(21) Numéro de dépôt: 02291727.2
(22) Date de dépôt: 09.07.2002
(51) Int. Cl.: C07C 17/10, C07C 17/21, C07C 17/20, C07C 19/12, C07C 19/08

(54) **Nouveau procédé pour la fabrication du 1,1,1-trifluoro-2,2-dichloroéthane**
Verfahren zur Herstellung von 1,1,1-Trifluoro-2,2-dichloroethan
Process for the preparation of 1,1,1-trifluoro-2,2-dichloroethane

(30) Priorité: 03.08.2001 FR 0110451
(43) Date de publication de la demande: 05.02.2003
(73) Titulaire: Atofina, 92800 Puteaux (FR)
(72) Inventeur: Boussand, Béatrice, 69110 Sainte Foy Les Lyon (FR); Jorda, Eric, 69005 Lyon (FR)

(56) Documents cités:
- EP-A- 0 346 612
- EP-A- 0 462 514
- EP-A- 0 526 908
- EP-A- 0 583 703
- EP-A- 0 638 535
- US-A- 5 723 700

## Description

La présente invention a pour objet un procédé de préparation du 1,1,1-trifluoro-2,2-dichloroéthane (F123) par chloration catalytique du 1,1,1-trifluoro-2-chloroéthane (F133a) en présence de fluorure d'hydrogène (HF). Elle concerne également l'application de ce procédé à un procédé de fabrication du pentafluoroéthane (F125).

Les composés F123 et F125 pouvant être utilisés comme substituts des perchlorofluorocarbures (CFC) dans le domaine des aérosols (agents propulseurs) et dans celui de la réfrigération, on recherche actuellement des procédés performants pour leur production industrielle.

Dans WO 95/16654 on décrit la mise en contact, à une température de 340 °C, du F133a avec du chlore et de l'HF en présence d'un catalyseur au chrome. Bien que la conversion du F133a dans cette réaction soit élevée, elle produit majoritairement, à cette température, du 1,1,1,2-tétrafluoroéthane (F134a). Ainsi, la sélectivité en F123 ne dépasse pas 15 %, ce qui ne permet pas d'envisager une production de ce composé dans des conditions industriellement acceptables.

Dans WO 94/11327 on mentionne la mise en contact, à des températures inférieures à 300 °C, du F133a avec du chlore et de l'HF en présence d'un catalyseur au chrome. Cette réaction est effectuée avec un très fort excès de chlore et d'HF, et conduit préférentiellement à la formation de F124 et de F125 ; ainsi la sélectivité en F123 reste inférieure à 8 % et le ratio série 110/série 120 est supérieur à 10%.

Dans EP-A-526 908 et EP-A-346 612, on propose de préparer du F123 par mise en contact de chlore avec le F133a, à une température se situant de préférence entre 350 et 450 °C, en l'absence ou en présence d'un catalyseur, cette chloration étant effectuée en l'absence d'HF.

Dans US-A-4 145 368, on propose un procédé consistant à faire réagir du chlore avec du F133a, puis à séparer le F123 du milieu réactionnel, à faire réagir le F113a résultant de cette séparation avec une nouvelle quantité de F133a, cette réaction étant conduite en phase vapeur et de préférence entre 350 et 425 °C, en présence d'un catalyseur tel qu'un oxyde de chrome.

Selon ce document, la sélectivité finale en F123 n'excède pas 29 %.

Dans EP-B-407 990 on propose la chloration de F133a en F123 par activation thermique ou catalytique, en phase liquide sous pression. La sélectivité en F123 peut aller de 67,9 à 83,4 %, la pression de réaction allant de 50 à 127 bars.

Dans EP-A-402 874, on propose de faire réagir le chlore avec le F133a entre 350 et 450 °C, en l'absence de catalyseur et de HF. Selon ce document, grâce à une combinaison particulière de conditions de température, de temps de contact et de rapport molaire des réactifs, on peut éliminer la production de F113a.

Dans US-A-5 414 166, on propose une chloration de F133a en présence d'hydrogène, entre 250 et 500 °C et de préférence entre 350 et 450 °C : la sélectivité en F123 peut aller de 65 à 92 %.

Dans US-A-5 723 700, on décrit une étape au cours de laquelle F133a, HF et Cl₂ réagissent, en présence d'un catalyseur de fluoration, entre 300 et 450 °C pour obtenir, essentiellement du F134a et des traces de F123.

L'invention a pour but de proposer un procédé de préparation de F123 par chloration de F133a, permettant d'obtenir une conversion du F133a et/ou une sélectivité en F123 améliorées.

L'invention a pour but également de proposer un procédé de préparation de F123 par chloration de F133a, pouvant être mis en oeuvre à une température relativement modérée.

Un autre but de l'invention est de proposer un procédé de préparation de F123 par chloration de F133a, pouvant être mis en oeuvre à pression atmosphérique ou sous pression modérée, par exemple n'excédant pas 25 bars.

Un autre but de l'invention est de proposer un procédé de préparation de F123 par chloration de F133a, donnant lieu à une sélectivité en F123 supérieure à 90 %.

Un autre but de l'invention est de proposer encore un tel procédé, donnant lieu à une sélectivité en F123 supérieure à 95 %.

Il a à présent été trouvé qu'au moins un des buts précités est atteint au moyen du procédé décrit ci-après.

La présente invention a donc pour objet un procédé de préparation de 1,1,1-trifluoro-2,2-dichloroéthane (F123) par mise en contact de 1,1,1-trifluoro-2-chloroéthane (F133a) avec du chlore, ledit procédé étant caractérisé en ce que ladite mise en contact est réalisée :
- en présence d'HF ;
- dans des conditions de température, de temps de contact et avec des rapports molaires Cl₂/F133a et HF/F133a tels que HF ne réagisse substantiellement pas avec le F133a et le F123 formé et favorise la sélectivité en F123 ; et
- en présence d'un catalyseur massique consistant en du fluorure d'aluminium ou en un mélange de fluorure d'aluminium et d'alumine, ou d'un catalyseur à base de fer, ou de fer et de nickel, supporté sur du fluorure d'aluminium ou sur un mélange de fluorure d'aluminium et d'alumine.

L'invention concerne plus particulièrement un procédé tel que HF ne réagisse substantiellement pas avec le F133a et le F123 pour donner des dérivés plus fluorés que le F133a, tels que le F124 ou le F134a.

Dans la mise en oeuvre du procédé selon l'invention, il convient de choisir des conditions opératoires telles que HF se comporte essentiellement comme diluant et/ou stabilisant de la réaction et des réactifs, et non pas en tant que réactif dans une réaction de fluoration. D'une manière générale, les conditions de température, les rapports molaires Cl₂/F133a et HF/F133a et le temps de contact peuvent être choisis à l'intérieur des fourchettes connues pour ce genre de réaction de chloration, et telles que rapportées par exemple ci-avant en référence aux documents concernant ladite réaction de chloration.

A titre purement illustratif, et qui ne saurait, de ce fait, limiter le domaine de l'invention, on indiquera ci-après des ordres de grandeur recommandés pour les conditions opératoires en question.

D'une manière générale, la température du milieu réactionnel est comprise entre 150 et 320 °C. Cette température est de préférence comprise entre 250 et 300 °C.

Le rapport molaire chlore/F133a peut être compris entre 0,01 et 0,50 et il est, de préférence, compris entre 0,1 et 0,3.

Le rapport molaire HF/F133a peut être généralement compris entre 0,5 et 2,5. Pour des raisons pratiques, entre autres liées à la séparation des produits en vue du recyclage des réactifs non consommés, et de l'HF, on choisit de préférence un rapport compris entre 0,8 et 1,2.

Le temps de contact entre F133a, le chlore et HF sur le catalyseur peut être compris entre 5 et 100 secondes ; il est recommandé d'avoir un temps de contact compris entre 10 et 60 secondes. Le temps de contact est ici calculé comme le rapport du volume apparent du catalyseur sur le débit volumique total des gaz alimentés au réacteur, dans les conditions de pression et de température de réaction.

Le catalyseur utilisable pour cette invention est un catalyseur consistant en du fluorure d'aluminium ou en un mélange de fluorure d'aluminium et d'alumine, sur lequel sont éventuellement déposés des oxydes, halogénures et/ou oxyhalogénures de fer, ou bien de fer et de nickel. Les catalyseurs consistant en du fluorure d'aluminium, seul ou en mélange avec l'alumine, sont décrits notamment dans EP 0609124. Les catalyseurs à base de fer, ou de fer et de nickel, sont préparés par imprégnation à sec de fluorure d'aluminium ou d'un mélange de fluorure d'aluminium et d'alumine soit par une solution de chlorure ferrique, soit par une solution contenant un mélange de chlorure de nickel et de chlorure ferrique. L'imprégnation est suivie d'une étape de séchage sous azote.

Lorsqu'on utilise un catalyseur à base de fer, la teneur en fer est généralement inférieure à 30 % en poids et est de préférence inférieure à 15 %. Lorsqu'on utilise un catalyseur à base de fer et de nickel, la teneur en nickel est inférieure à 20 % en poids, de préférence inférieure à 15 %.

Préalablement à la réaction de chloration, le catalyseur pourra être conditionné si nécessaire par un traitement thermique en présence de Cl₂ et/ou HF, par exemple en suivant la méthode décrite dans EP-B-0 609 124.

Ainsi qu'il a été précisé, le procédé conforme à l'invention consiste notamment à effectuer la mise en contact du chlore avec le F133a, en présence d'HF et d'un catalyseur, dans des conditions telles que HF ne réagisse substantiellement pas avec le F133a et le 123 formé pour donner des dérivés plus fluorés. Ces conditions sont avantageusement choisies à l'intérieur des zones de température, rapports molaires, temps de contact indiqués précédemment et il appartiendra à l'homme de l'art de choisir les conditions exactes d'une réaction compte tenu du résultat escompté. Ainsi, à partir du moment où le paramètre de température aura été choisi, par exemple 280 °C, il pourra être avantageux de réduire le rapport molaire Cl₂/F133a, par exemple autour de 10 % pour obtenir la meilleure sélectivité en série 120 au détriment de la série 110. De même, il conviendra, pour une certaine température et un certain taux de chlore, de choisir le temps de contact permettant d'associer le taux de conversion du F133a convenable et une bonne sélectivité en F123. De même encore, le rapport molaire HF/F133a pourra être choisi par exemple en fonction des valeurs désirées ou acceptables pour le rapport molaire série 110/série 120. Ainsi qu'il a été indiqué, ce rapport HF/F133a peut aller généralement de 0,5 à 2,5 compte tenu des autres conditions de réaction (température, temps de contact, rapport molaire Cl₂/F133a), mais, de préférence, ce rapport molaire HF/F133a se situe aux alentours de 1, par exemple entre 0,8 et 1,2.

Les indications qui précèdent montrent à l'évidence que les valeurs recommandées pour les conditions opératoires ont un rôle essentiellement informatif, sachant que l'on ne sortirait pas du cadre de l'invention en retenant, pour l'un ou l'autre des paramètres de la réaction, des valeurs situées au-delà ou en deçà des valeurs indiquées plus haut, pour autant que de telles modifications opératoires n'impliquent pas une réaction de HF avec le F133a conduisant à la formation de quantités substantielles de dérivés plus fluorés.

La réaction de chloration peut être conduite en phase gazeuse, en lit fixe ou en lit fluide, en batch, ou, de préférence, en continu, avec possibilité de recyclage dans le réacteur des réactifs non transformés et de l'HF. L'acide chlorhydrique formé lors de la réaction est séparé de préférence avant le recyclage. Le F123 récupéré peut être purifié par distillation selon la pureté désirée.

Le chlore peut être introduit dans le réacteur pur ou dilué dans un gaz inerte tel que l'azote. Les matériaux utilisés pour la construction de l'installation doivent être compatibles avec la présence de chlore et d'hydracides tels que HCl et HF ; ils peuvent être choisis par exemple en "Hastelloy" ou en "Inconel" qui sont résistants aux milieux corrosifs contenant ces hydracides.

La réaction de chloration selon l'invention peut être menée à la pression atmosphérique ou à une pression supérieure à cette dernière. Pour des raisons pratiques, on opère généralement dans une zone allant de 0 à 25 bars relatifs et de préférence entre 0 et 15 bars relatifs.

Dans des conditions opératoires susceptibles d'encrasser le catalyseur, il peut être judicieux d'introduire avec les réactifs, de l'oxygène à faible teneur. Cette teneur peut varier selon les conditions opératoires entre 0,02 et 5 % par rapport aux réactifs organiques (pourcentage molaire). L'oxygène pourra être introduit de manière continue ou séquentielle.

Le procédé conforme à l'invention permet de préparer du F123 à partir du F133a, dans des conditions de température et de pression modérées, et avec une excellente sélectivité en F123.

Le 1,1,1-trifluoro-2-chloroéthane (F133a) de départ peut lui-même être obtenu par application de procédés maintenant bien connus. Le F133a peut notamment être préparé par fluoration du trichloroéthylène (par exemple en suivant la méthode préconisée par Mc Bee et al., Ind. Eng. Chem. 39, 409-412), par fluoration du F132b, par fluoration du F130a, par hydrogénolyse du F113a, par fluoration du F1122 (CF₂ = CHCl).

Dans l'invention, on donne la préférence au F133a obtenu par fluoration du trichloroéthylène.

A ce titre, l'invention a également pour objet un procédé de préparation de F123, à partir de trichloroéthylène, ledit procédé comprenant :
a) une étape de fluoration du trichloroéthylène, dans une réaction, en phase liquide ou phase gazeuse, en présence d'un catalyseur et sous une pression conduisant après séparation d'HCl et des lourds, à un mélange de F133a accompagné d'HF, entraîné sous forme azéotropique ;
b) une étape de chloration du F133a, par mise en contact dudit F133a avec du chlore, en présence de HF et d'un catalyseur, dans des conditions de température, de temps de contact et avec des rapports Cl₂/F133a et HF/F133a tels que HF ne réagisse substantiellement pas avec le F133a et le F123 formé.

Dans la phase a), pour un procédé en phase liquide, on utilise de préférence un catalyseur à base de sels d'antimoine et on opère avantageusement sous une pression d'au moins 10 bars absolus. On peut également faire réagir le trichloroéthylène avec HF en présence d'oxyde de chrome ou d'oxyfluorure de chrome dans un procédé en phase gazeuse.

Le F123 peut être utilisé tel quel, par exemple comme agent propulseur en réfrigération et dans la fabrication de mousses, où il remplace avantageusement le F11 du fait de son innocuité sur l'ozone de la stratosphère. De ce fait, le procédé conforme à l'invention qui, par les résultats auxquels il conduit, est exploitable au niveau industriel, est particulièrement intéressant, tant au départ de trichloroéthylène qu'à celui de F133a.

Le F123 peut aussi subir une fluoration supplémentaire et conduire ainsi au F125 (pentafluoroéthane). Cette réaction peut être conduite selon divers procédés maintenant connus : d'une manière générale, cette étape comprend la mise en contact du F123 (seul ou en mélange avec d'autres composés de la série 120) avec de l'HF en présence d'un catalyseur de fluoration pour obtenir du F125.

Cette étape peut être réalisée en phase vapeur, et le catalyseur peut être choisi parmi les catalyseurs dont l'emploi est décrit par exemple, dans EP-B-609 124 ou dans les références auxquelles ce brevet renvoie, ledit brevet étant incorporé ici notamment pour les conditions recommandées pour cette réaction.

L'invention a donc encore pour objet un procédé de préparation de pentafluoroéthane (F125), ledit procédé comprenant :
a) une étape de fluoration de trichloroéthylène telle que décrite précédemment et aboutissant notamment à du F133a ;
b) une étape de chloration du F133a, telle que décrite précédemment et aboutissant notamment à du F123 ;
c) une étape de fluoration de F123, par mise en contact de F123 avec HF, en présence d'un catalyseur, avec ou sans recyclage de F124, la fluoration du F124 pouvant faire l'objet d'une étape séparée.

Dans cette étape, le catalyseur est, avantageusement, un catalyseur mixte composé d'oxydes, d'halogénures et/ou d' oxyhalogénures de nickel et de chrome tel que décrit dans EP-B-609 124. On peut également utiliser les catalyseurs à base d'oxyde ou d'oxyfluorure de chrome ou à base d'alumine ou de fluorure d'aluminium, éventuellement dopé par un métal tel que zinc, nickel ou fer. De tels catalyseurs sont décrits par exemple dans EP-502 605 ou WO 93/16 798. On peut encore utiliser un catalyseur du type chrome/charbon tel que décrit par exemple dans EP-A-456 552.

Pour cette étape, on utilise de préférence un catalyseur mixte décrit ci-dessus déposé sur un support constitué de fluorure d'aluminium ou d'un mélange de fluorure d'aluminium et d'alumine.

La température de cette réaction de fluoration peut être comprise entre 250 et 470 °C et est de préférence comprise entre 280 et 410 °C. Le temps de contact entre HF et F123 peut être compris entre 3 et 100 s, de préférence entre 5 et 30 s. Le rapport molaire HF/F123 peut aller de 1/1 à 20/1 et de préférence de 2/1 à 9/1.

Bien que la réaction puisse être conduite à pression atmosphérique, on préfère opérer sous légère pression, par exemple n'excédant pas 10 bars absolus et même inférieure à 5 bars absolus.

Le F125 obtenu peut être ensuite purifié par exemple par application des méthodes décrites dans FR-2 758 137 ou WO 95/21147 dont le contenu est incorporé ici par référence.

Le procédé de préparation de F125 à partir de trichloroéthylène, qui constitue également un objet de l'invention, peut être mis en oeuvre en continu dans une installation telle que présentée schématiquement par l'une ou l'autre des figures jointes.

Ces figures illustrent un schéma d'ensemble des trois étapes I, II et III du procédé en question.

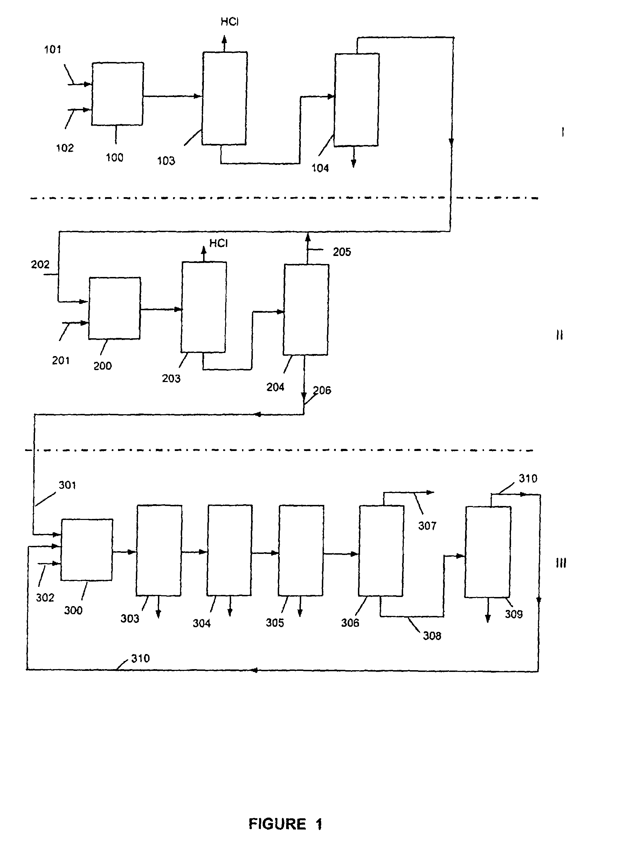

L'installation comprend notamment (voir figure 1) :

### Etape I

- un réacteur (100), contenant le catalyseur ;
- des arrivées de trichloroéthylène (101) et d'HF (102) ;
- une colonne de distillation d'HCl (103) ;
- une colonne de séparation de F133a + HF des lourds (104) :

### Etape II

- un réacteur de chloration (200) alimenté par :
   ■ du F133a et de l'HF (202) ;
   ■ du chlore (201 ) ;
- une colonne de séparation d'HCl (203) ;
- une colonne (204) de séparation du F123 brut (206) du F133a n'ayant pas réagi (+ notamment HF azéotropique + Cl₂ n'ayant pas réagi) qui sont recyclés (205) ;
- un moyen de soutirage de l'HF excédentaire.

### Etape III

- un réacteur de fluoration (300), alimenté en F123 brut (206/301) provenant de la colonne (204) de l'étape précédente, en HF (302) et éventuellement en F124 brut (310) recyclé depuis la colonne (309) ;
- à la sortie du réacteur (300), les dispositifs de traitement des gaz réactionnels (colonnes 303, 304 et 305) destinés à récupérer l'HCl sous produit par la réaction et l'HF non transformé, et à neutraliser les composés fluorocarbonés avant leur distillation ;
- une colonne (306) permettant ensuite d'extraire en tête le F125 (307), le pied (308) étant ensuite distillé sur la colonne (309) pour obtenir un mélange F124 + F123 (310) purifié de son contenu en lourds, ledit mélange étant ensuite recyclé en réaction (300) pour y être fluoré en F125.

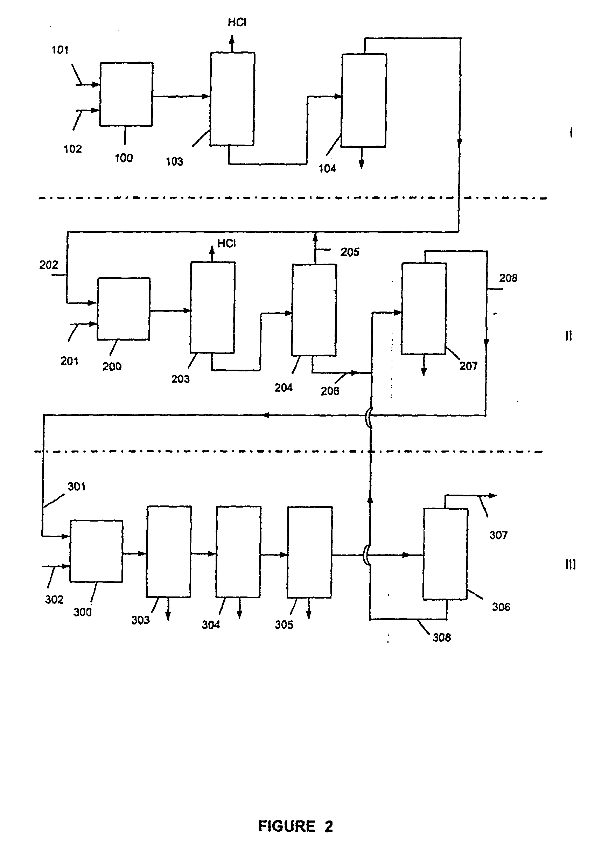

Une option avantageuse de ce schéma est représente sur la figure 2, dans laquelle une colonne (207) permet de purifier à la fois le F123 brut (206) issu de la colonne (204) de l'étape II et le mélange brut F124 + F123 issu du pied de la colonne (306) de l'étape III : dans cette option, qui permet d'alimenter la réaction (300) en produits plus propres, la colonne (309) n'a plus lieu d'exister.

Bien entendu, l'installation industrielle comprend des dispositifs complémentaires dont l'usage est connu (purge, évaporateurs, surchauffeur, décanteurs).

L'invention concerne donc également une installation de production de F125 à partir de trichloroéthylène, et qui comprend au moins la succession de dispositifs tels que représentés sur la figure.

L'invention sera maintenant illustrée par les exemples suivants, qui ne sont donnés qu'à titre purement indicatif.

### Exemple 1 : Préparation de F123 par chloration de 133a en présence d'HF et d'un catalyseur à base de fer.

Dans un tube en Inconel de diamètre interne 21 mm, 75 cm³ de catalyseur à base de fer déposé sur alumine fluorée ayant une teneur en Fe de 12 % en poids sont introduits.

Le débit d'HF est ajusté à 0,91 mol/h et la température à 270°C. Ensuite, un mélange Cl₂/N₂ à 15 % molaire de chlore est introduit dans le réacteur avec un débit de 0,91 mol/h. Enfin, CF₃-CH₂Cl est introduit dans le réacteur avec un débit de 0,90 mol/h et la pression totale de réaction est régulée à 15 bars.

Après 24h de réaction, un échantillon gazeux est prélevé pour analyse en chromatographie en phase gazeuse. Un autre échantillon est prélevé après avoir débarassé le flux issu du réacteur de l'HF et du chlore par bullage dans des flacons laveurs à eau et soude/sulfite puis l'avoir séché sur CaCl₂. Il est de même analysé par chromatographie en phase gazeuse.

La conversion de F133a est de 7,3 %, pour une sélectivité en F123 de 96,9 %. Le ratio série 110/série 120 est de 2,9 %.

### Exemples 2 à 4 : Préparation de F123 par chloration de 133a en présence d'HF et d'un catalyseur à base de fer.

Suivant le même protocole que l'exemple 1, différentes conditions ont été testées. Les conditions testées et résultats obtenus sont rassemblés dans le tableau ci-dessous :

| **Conditions** | **Exemple 2** | **Exemple 3** | **Exemple 4** |
|---|---|---|---|
| Température (°C) | 270 | 280 | 280 |
| RM Cl₂/F133a | 0,25 | 0,15 | 0,25 |
| RM HF/F133a | 1,1 | 1,1 | 1,2 |
| Tc (s) | 27 | 32 | 27 |

| *Résultats* | | | |
|---|---|---|---|
| Conversion F133a % | 9,0 | 9,4 | 12,6 |
| Sélectivité F123 % | 96,4 | 94,8 | 93,7 |
| Ratio série 110/120 % | 3,4 | 3,9 | 5,7 |

### Exemples 5 à 7 : Préparation de F123 par chloration de 133a en présence d'HF et d'alumine fluorée comme catalyseur.

Suivant le même protocole que l'exemple 1, mais en remplaçant le catalyseur par une alumine fluorée; différentes conditions ont été testées.

Les conditions testées et résultats obtenus sont rassemblés dans le tableau ci-dessous :

| **Conditions** | **Exemple 5** | **Exemple 6** | **Exemple 7** |
|---|---|---|---|
| Température (°C) | 270 | 280 | 280 |
| RM Cl₂/F133a | 0,23 | 0,18 | 0,27 |
| RM HF/F133a | 1,0 | 1,2 | 1,0 |
| Tc (s) | 26 35 | | 29 |

| *Résultats* | | | |
|---|---|---|---|
| Conversion F133a % | 7,9 | 10,2 | 11,9 |
| Sélectivité F123 % | 98,4 | 95,9 | 96,4 |
| Ratio série 110/120 % | 1,6 | 4,1 | 3,4 |

### Exemple 8 (comparatif) : Préparation de F123 par chloration de 133a en présence d'alumine fluorée comme catalyseur et sans HF.

Dans un tube en Inconel de diamètre interne 21 mm, 75 cm³ de catalyseur (alumine fluorée) sont introduits. Le catalyseur est traité pendant 15h avec 1 mol/h d'HF anhydre à 350°C et pression atmosphérique.

Préalablement à la réaction, le débit d'HF est arrêté, la température descendue à 280°C et un débit d'azote de 1,02 mol/h est introduit dans le réacteur. Ensuite, un mélange Cl₂/N₂ à 15 % molaire de chlore est introduit dans le réacteur avec un débit de 1,02 mol/h. Enfin, CF₃-CH₂Cl est introduit dans le réacteur avec un débit de 1,1 mol/h et la pression totale de réaction est régulée à 15 bars.

Après 24h de réaction, un échantillon gazeux est. prélevé pour analyse en chromatographie en phase gazeuse. Un autre échantillon est prélevé après avoir débarrassé le flux issu du réacteur de l'HF et du chlore par bullage dans des flacons laveurs à eau et soude/sulfite puis l'avoir séché sur CaCl₂. Il est de même analysé par chromatographie en phase gazeuse.

La conversion de F133a est de 9,5 %, pour une sélectivité en F123 de 76,7 %. Le ratio série 110/série 120 est de 23,5 %.

### Exemple 9 (comparatif) : Préparation de F123 par chloration de 133a en présence d'alumine fluorée comme catalyseur et sans HF :

Un essai a été réalisé suivant le même protocole que l'exemple 8 mais dans les conditions suivantes : température = 280°C, rapport molaire Cl₂/F133a = 0,27, rapport molaire N₂/F133a = 1, temps de contact = 23 s.

La conversion du F133a est alors de 17 %, pour une sélectivité en F123 de 53 %. Le ratio série 110/série 120 est de 70 %.

### Exemple 10 (comparatif) : Préparation de F123 par chloration de 133a sans HF et sans catalyseur.

Dans un tube en Inconel vide de diamètre interne 21 mm, chauffé à 280°C, un débit d'azote de 0,94 mol/h est introduit ainsi qu'un mélange Cl₂/N₂ à 15 % molaire de chlore avec un débit de 0,95 mol/h. Enfin, CF₃-CH₂Cl est introduit dans le réacteur avec un débit de 0,88 mol/h et la pression totale de réaction est régulée à 15 bars.

Après 24h de réaction, un échantillon gazeux est prélevé pour analyse en chromatographie en phase gazeuse. Un autre échantillon est prélevé après avoir débarrassé le flux issu du réacteur de l'HF et du chlore par bullage dans des flacons laveurs à eau et soude/sulfite puis l'avoir séché sur CaCl₂. Il est de même analysé par chromatographie en phase gazeuse.

La conversion du F133a est de 2,5 %, pour une sélectivité en F123 de 91 %.

Le ratio série 110/série 120 est de 8,5 %.

### Exemple 11 (comparatif) : Préparation de F123 par chloration de 133a sans HF et sans catalyseur.

Un essai a été réalisé suivant le même protocole que l'exemple 10 mais dans les conditions suivantes : température = 280°C, rapport molaire Cl₂/F133a = 0,25, rapport molaire N₂/F133a = 1, débit de F133a = 0,93 mol/h.

La conversion du F133a est alors de 2,8 %, pour une sélectivité en F123 de 87 %. Le ratio série 110/série 120 est de 13 %.

### Exemple 12 (comparatif) : Préparation de F123 par chloration de 133a en présence d'HF et d'un catalyseur Ni-Cr supporté sur alumine fluorée

Dans un tube en Inconel de diamètre interne 21 mm, on introduit 75 cm³ de catalyseur Ni-Cr supporté sur alumine fluorée ayant une teneur en Ni de 6 % poids et une teneur en Cr de 6 % poids (préparé comme décrit dans le brevet FR 2669022). Le catalyseur est traité pendant 15h avec 1 mol/h d'HF anhydre à 350°C et pression atmopshérique.

Préalablement à la réaction, le débit d'HF est ajusté à 1,2 mol/h et la température à 280°C. Ensuite, un mélange Cl₂/N₂ à 15 % molaire de chlore est introduit dans le réacteur avec un débit de 1,56 mol/h. Enfin CF₃-CH₂Cl est introduit dans le réacteur avec un débit de 1,18 mol/h et la pression totale de réaction est régulée à 15 bars.

Après 24h de réaction, un échantillon gazeux est prélevé pour analyse en chromatographie en phase gazeuse. Un autre échantillon est prélevé après avoir débarrassé le flux issu du réacteur de l'HF et du chlore par bullage dans les flacons laveurs à eau et soude/sulfite puis l'avoir séché sur CaCl₂. Il est de même analysé par chromatographie en phase gazeuse.

La conversion du F133a est de 12,8 % pour une sélectivité en F123 de 75,3 %. Le ratio série 110/série 120 est de 8,2 %.

### Exemples 13 et 14 (comparatifs): Préparation de F123 par chloration de 133a en présence d'HF et d'un catalyseur Ni-Cr supporté sur alumine fluorée.

Suivant le même protocole que l'exemple 12, différentes conditions ont été testées. Les résultats sont indiqués dans le tableau ci-dessous :

| **Conditions** | **Exemple 13** | **Exemple 14** |
|---|---|---|
| Température (°C) | 270 | 280 |
| RM Cl2/F133a | 0,28 | 0,28 |
| RM HF/F133a | 1,0 | 0,9 |
| Tc (s) | 32 | 31 |

| *Résultats* | | |
|---|---|---|
| Conversion F133a % | 16,9 | 21 |
| Sélectivité F123 % | 73,7 | 74 |
| ratio série 110/série 120 % | 13,1 | 12,9 |

## Revendications

1. Procédé de préparation de 1,1,1-trifluoro-2,2-dichloroéthane (F123) par mise en contact de 1,1,1-trifluoro-2-chloroéthane (F133a) avec du chlore, ledit procédé étant **caractérisé en ce que** la mise en contact est réalisée :
- en présence d'HF ;
- dans des conditions de température, de temps de contact et avec des rapports molaires Cl₂/F133a et HF/F133a tels que HF ne réagisse substantiellement pas avec le F133a et le F123 formé et favorise la sélectivité en F123 ; et
- en présence d'un catalyseur massique consistant en du fluorure d'aluminium ou en un mélange de fluorure d'aluminium et d'alumine, ou d'un catalyseur à base de fer, ou de fer et de nickel, supporté sur du fluorure d'aluminium ou sur un mélange de fluorure d'aluminium et d'alumine.

2. Procédé selon la revendication 1, dans lequel la température est comprise entre 150 et 320 °C.

3. Procédé selon la revendication 2, dans lequel la température est comprise entre 250 et 300 °C.

4. Procédé selon l'une quelconque des revendications 1 à 3, dans lequel le rapport molaire Cl₂/F133a est compris entre 0,01 et 0,50.

5. Procédé selon la revendication 4, dans lequel le rapport molaire Cl₂/F133a est compris entre 0,1 et 0,3.

6. Procédé selon l'une quelconque des revendications 1 à 5, dans lequel le rapport molaire HF/F133a est compris entre 0,5 et 2,5.

7. Procédé selon la revendication 6, dans lequel le rapport molaire HF/F133a est compris entre 0,8 et 1,2.

8. Procédé selon l'une quelconque des revendications 1 à 7, dans lequel le temps de contact entre F133a, Cl₂, et d'HF sur le catalyseur est compris entre 5 et 100 secondes.

9. Procédé selon la revendication 8, dans lequel le temps de contact est compris entre 10 et 60 secondes.

10. Procédé selon l'une quelconque des revendications 1 à 9, dans lequel le catalyseur est un catalyseur massique consistant en du fluorure d'aluminium ou en un mélange de fluorure d'aluminium et d'alumine.

11. Procédé selon l'une quelconque des revendications 1 à 9, dans lequel le catalyseur est un catalyseur à base de fer supporté sur du fluorure d'aluminium ou sur un mélange de fluorure d'aluminium et d'alumine.

12. Procédé selon la revendication 11, dans lequel la teneur en fer est inférieure 30 % en poids, de préférence à 15 %.

13. Procédé selon l'une quelconque des revendications 1 à 9 ou selon la revendication 11 ou 12, dans lequel le catalyseur est un catalyseur à base de fer et de nickel supporté sur du fluorure d'aluminium ou sur un mélange de fluorure d'aluminium et d'alumine.

14. Procédé selon la revendication 13, dans lequel la teneur en nickel du catalyseur est inférieure à 20 % en poids, de préférence inférieure à 15 %.

15. Procédé selon l'une quelconque des revendications 1 à 14, **caractérisé en ce qu'**il est mis en oeuvre en continu.

16. Procédé selon l'une quelconque des revendications 1 à 15, dans lequel le F133a de départ est obtenu par fluoration du trichloroéthylène.

17. Procédé selon la revendication 16, dans lequel la fluoration du trichloroéthylène est réalisée en phase liquide sous pression, en présence d'un catalyseur à base de sels d'antimoine ou en phase gazeuse en présence d'un catalyseur à base d'oxyde de chrome ou d'oxyfluorure de chrome.

18. Procédé de préparation de pentafluoroéthane (F125) par fluoration de F123 **caractérisé en ce que** le dit F123 est preparé selon l'une quelconque des revendications 1 à 17, par mise an contact de F133a avec du chlore.

19. Procédé selon la revendication 18, dans lequel on met le F123 en contact avec HF en présence d'un catalyseur tel que décrit dans la revendication 1.

20. Procédé selon la revendication 18, dans lequel le F133a de départ est obtenu selon l'une des revendications 16 ou 17, ledit procédé comprenant :
- une étape I de fluoration de trichloréthylène en F133a
- une étape II de chloration de F133a en F123
- une étape III de fluoration de F123 en F125.

21. Installation permettant la mise en oeuvre du procédé selon la revendication 20, **caractérisée en ce qu'**elle comprend : (fig. 1)
pour l'étape I :
- un réacteur (100), contenant le catalyseur ;
- des arrivées de trichloroéthylène (101) et d'HF (102) ;
- une colonne de distillation d'HCl (103) ;
- une colonne de séparation de F133a + HF des lourds (104).
pour l'étape II :
- un réacteur de chloration (200) alimenté par :
■ du F133a et de l'HF (202),
■ du chlore (201) ;
- une colonne de séparation d'HCl (203) ;
- une colonne (204) de séparation du F123 brut (206) du F133a n'ayant pas réagi (+ notamment HF azéotropique + Cl₂ n'ayant pas réagi) qui sont recyclés (205) ;
- un moyen de soutirage de l'HF excédentaire.
pour l'étape III :
- un réacteur de fluoration (300), alimenté en F123 brut (206/301) provenant de la colonne (204) de l'étape précédente, en HF (302) et éventuellement en F124 brut (310) recyclé depuis la colonne (309) ;
- à la sortie du réacteur (300), les dispositifs de traitement des gaz réactionnels (colonnes 303, 304 et 305) destinés à récupérer l'HCl sous produit par la réaction et l'HF non transformé, et à neutraliser les composés fluorocarbonés avant leur distillation ;
- une colonne (306) permettant ensuite d'extraire en tête le F125 (307), le pied (308) étant ensuite distillé sur la colonne (309) pour obtenir un mélange F124 + F123 (310) purifié de son contenu en lourds, ledit mélange étant ensuite recyclé en réaction (300) pour y être fluoré en F125.

22. Installation selon la revendication 21, **caractérisée en ce qu'**elle comprend (fig. 2) une colonne (207) permettant de purifier à la fois le F123 brut (206) issu de la colonne (204) de l'étape II et le mélange brut F124 + F123 issu du pied de la colonne (306) de l'étape III.

## Patentansprüche

1. Verfahren zur Herstellung von 1,1,1-Trifluor-2,2-dichlorethan (F123) durch Inkontaktbringen von 1,1,1-Trifluor-2-chlorethan (F133a) mit Chlor, **dadurch gekennzeichnet, daß** man das Inkontaktbringen
- in Gegenwart von HF;
- unter solchen Temperatur- und Kontaktzeitbedingungen und mit solchen Cl₂/F133a- und HF/F133a-Molverhältnissen, daß das HF praktisch nicht mit dem F133a und dem gebildeten F123 reagiert und die F123-Selektivität fördert; und
- in Gegenwart eines Vollkatalysators aus Aluminiumfluorid oder einer Mischung aus Aluminiumfluorid und Aluminiumoxid oder eines Katalysators auf Basis von Eisen oder Eisen und Nickel mit Aluminiumfluorid oder einer Mischung aus Aluminiumfluorid und Aluminiumoxid als Träger
durchführt.

2. Verfahren nach Anspruch 1, bei dem die Temperatur zwischen 150 und 320°C liegt.

3. Verfahren nach Anspruch 2, bei dem die Temperatur zwischen 250 und 300°C liegt.

4. Verfahren nach einem der Ansprüche 1 bis 3, bei dem das Cl₂/F133a-Molverhältnis zwischen 0,01 und 0,50 liegt.

5. Verfahren nach Anspruch 4, bei dem das Cl₂/F133a-Molverhältnis zwischen 0,1 und 0,3 liegt.

6. Verfahren nach einem der Ansprüche 1 bis 5, bei dem das HF/F133a-Molverhältnis zwischen 0,5 und 2,5 liegt.

7. Verfahren nach Anspruch 6, bei dem das HF/F133a-Molverhältnis zwischen 0,8 und 1,2 liegt.

8. Verfahren nach einem der Ansprüche 1 bis 7, bei dem die Zeit für den Kontakt zwischen F133a, Cl₂ und HF an dem Katalysator zwischen 5 und 100 Sekunden liegt.

9. Verfahren nach Anspruch 8, bei dem die Kontaktzeit zwischen 10 und 60 Sekunden liegt.

10. Verfahren nach einem der Ansprüche 1 bis 9, bei dem es sich bei dem Katalysator um einen Vollkatalysator aus Aluminiumfluorid oder einer Mischung aus Aluminiumfluorid und Aluminiumoxid handelt.

11. Verfahren nach einem der Ansprüche 1 bis 9, bei dem es sich bei dem Katalysator um einen Katalysator auf Basis von Eisen mit Aluminiumfluorid oder einer Mischung aus Aluminiumfluorid und Aluminiumoxid als Träger handelt.

12. Verfahren nach Anspruch 11, bei dem der Eisengehalt unter 30 Gew.-% und vorzugsweise unter 15% liegt.

13. Verfahren nach einem der Ansprüche 1 bis 9, 11 oder 12, bei dem es sich bei dem Katalysator um einen Katalysator auf Basis von Eisen und Nickel mit Aluminiumfluorid oder einer Mischung aus Aluminiumfluorid und Aluminiumoxid als Träger handelt.

14. Verfahren nach Anspruch 13, bei dem der Nickelgehalt des Katalysators unter 20 Gew.-% und vorzugsweise unter 15% liegt.

15. Verfahren nach einem der Ansprüche 1 bis 14, **dadurch gekennzeichnet, daß** man es kontinuierlich betreibt.

16. Verfahren nach einem der Ansprüche 1 bis 15, bei dem man das F133a-Edukt durch Fluorierung von Trichlorethylen erhält.

17. Verfahren nach Anspruch 16, bei dem man die Fluorierung des Trichlorethylens in der Flüssigphase unter Druck in Gegenwart eines Katalysators auf Basis von Antimonsalzen oder in der Gasphase in Gegenwart eines Katalysators auf Basis von Chromoxid oder Chromoxidfluorid durchführt.

18. Verfahren zur Herstellung von Pentafluorethan (F125) durch Fluorierung von F123, **dadurch gekennzeichnet, daß** man das F123 gemäß einem der Ansprüche 1 bis 17 durch Inkontaktbringen von F133a mit Chlor herstellt.

19. Verfahren nach Anspruch 18, bei dem man das F123 in Gegenwart eines Katalysators gemäß Anspruch 1 mit dem HF in Kontakt bringt.

20. Verfahren nach Anspruch 18, bei dem man das F133a-Edukt gemäß Anspruch 16 oder 17 erhält und:
- in einem Schritt I Trichlorethylen zu F133a fluoriert,
- in einem Schritt II F133a zu F123 chloriert,
- in einem Schritt III F123 zu F125 fluoriert.

21. Anlage zur Durchführung des Verfahrens nach Anspruch 20, **dadurch gekennzeichnet, daß** sie folgendes aufweist (Fig. 1):
für Schritt I:
- einen Reaktor (100), der den Katalysator enthält;
- Einlässe für Trichlorethylen (101) und HF (102);
- eine HCl-Destillationssäule (103);
- eine Säule (104) zur Trennung von F133a + HF von Schwersiedern;
für Schritt II:
- einen Chlorierungsreaktor (200), der mit
■ F133a und HF (202),
■ Chlor (201) gespeist wird;
- eine Säule (203) zur Abtrennung von HCl;
- eine Säule (204) zur Abtrennung von rohem F123 (206) von nicht umgesetztem F133a (+ insbesondere azeotropem HF + nicht umgesetztem Cl₂), die zurückgeführt werden (205);
- eine Einrichtung zum Abziehen von überschüssigem HF;
für Schritt III:
- einen Fluorierungsreaktor (300), der mit rohem F123 (206/301) aus der Säule (204) des vorhergehenden Schritts, HF (302) und gegebenenfalls aus der Säule (309) zurückgeführtem rohem F124 (310) gespeist wird,
- am Ausgang des Reaktors (300) Vorrichtungen zur Behandlung der Reaktionsgase (Säulen 303, 304 und 305) zur Rückgewinnung von HCl-Reaktionsnebenprodukt und nicht umgewandeltem HF und zur Neutralisierung der Fluorkohlenwasserstoffverbindungen vor ihrer Destillation;
- eine Säule (306), aus der dann das F125 (307) über Kopf abgezogen werden kann, wobei das Sumpfprodukt (308) danach in der Säule (309) destilliert wird, wobei man ein von seinem Gehalt an Schwersiedern befreites Gemisch aus F124 + F123 (310) erhält, welches danach zwecks Fluorierung zu F125 in den Reaktor (300) zurückgeführt wird.

22. Anlage nach Anspruch 21, **dadurch gekennzeichnet, daß** sie eine Säule (207) zur gleichzeitigen Reinigung des rohen F123 (206) aus der Säule (204) von Schritt II und des rohen Gemischs von F124 + F123 aus dem Sumpf der Säule (306) von Schritt III aufweist (Fig. 2).

## Claims

1. Process for the preparation of 1,1,1-trifluoro-2,2-dichloroethane (F123) by bringing 1,1,1-trifluoro-2-chloroethane (F133a) into contact with chlorine, the said process being **characterized in that** the contacting operation is carried out:
- in the presence of HF;
- under conditions of temperature and of contact time and with Cl₂/F133a and HF/F133a molar ratios such that HF does not substantially react with the F133a and the F123 formed and promotes the selectivity for F123; and
- in the presence of a bulk catalyst consisting of aluminium fluoride or of a mixture of aluminium fluoride and of alumina, or of a catalyst based on iron, or on iron and on nickel, supported on aluminium fluoride or on a mixture of aluminium fluoride and of alumina.

2. Process according to Claim 1, in which the temperature is between 150 and 320°C.

3. Process according to Claim 2, in which the temperature is between 250 and 300°C.

4. Process according to any one of Claims 1 to 3, in which the Cl₂/F133a molar ratio is between 0.01 and 0.50.

5. Process according to Claim 4, in which the Cl₂/F133a molar ratio is between 0.05 and 0.15.

6. Process according to any one of Claims 1 to 5, in which the HF/F133a molar ratio is between 0.5 and 2.5.

7. Process according to Claim 6, in which the HF/F133a molar ratio is between 0.8 and 1.2.

8. Process according to any one of Claims 1 to 7, in which the time for contact between F133a, Cl₂ and HF over the catalyst is between 5 and 100 seconds.

9. Process according to Claim 8, in which the contact time is between 10 and 60 seconds.

10. Process according to any one of Claims 1 to 9, in which the catalyst is a bulk catalyst consisting of aluminium fluoride or of a mixture of aluminium fluoride and of alumina.

11. Process according to any one of Claims 1 to 9, in which the catalyst is a catalyst based on iron supported on aluminium fluoride or on a mixture of aluminium and of alumina.

12. Process according to Claim 11, in which the iron content is less than 30% by weight, preferably less than 15%.

13. Process according to any one of Claims 1 to 9 or according to Claim 11 or 12, in which the catalyst is a catalyst based on iron and on nickel supported on aluminium fluoride or on a mixture of aluminium fluoride and of alumina.

14. Process according to Claim 13, in which the nickel content of the catalyst is less than 20% by weight, preferably less than 15%.

15. Process according to any one of Claims 1 to 14, **characterized in that** it is carried out continuously.

16. Process according to any one of Claims 1 to 15, in which the starting F133a is obtained by fluorination of trichloroethylene.

17. Process according to Claim 16, in which the fluorination of the trichloroethylene is carried out in the liquid phase under pressure in the presence of a catalyst based on antimony salts or in the gas phase in the presence of a catalyst based on chromium oxide or on chromium oxyfluoride.

18. Process for the preparation of pentafluoroethane (F125) by fluorination of F123, **characterized in that** the said F123 is prepared, according to any one of Claims 1 to 17, by bringing F133a into contact with chlorine.

19. Process according to Claim 18, in which the F123 is brought into contact with HF in the presence of a catalyst as described in Claim 1.

20. Process according to Claim 18, in which the starting F133a is obtained according to either of Claims 16 and 17, the said process comprising:
- a stage I of fluorination of trichloroethylene to F133a
- a stage II of chlorination of F133a to F123
- a stage III of fluorination of F123 to F125.

21. Plant making possible the implementation of the process according to Claim 20, **characterized in that** it comprises: (Fig. 1)
for Stage I:
- a reactor (100) comprising the catalyst;
- trichloroethylene (101) and HF (102) inlets;
- an HCl distillation column (103);
- a column for separation of F133a + HF from the heavy products (104);
for Stage II:
- a chlorination reactor (200) fed with:
■ F133a and HF (202);
■ chlorine (201);
- an HCl separation column (203);
- a column (204) for separation of crude F123 (206) from unreacted F133a (+ in particular azeotropic HF + unreacted Cl₂), which are recycled (205);
- a means for withdrawing excess HF;
for Stage III:
- a fluorination reactor (300), fed with crude F123 (206/301) originating from the column (204) of the preceding stage, with HF (302) and optionally with crude F124 (310) recycled from the column (309);
- at the outlet of the reactor (300), devices for treating the reaction gases (columns 303, 304 and 305) intended to recover the HCl reaction by-product and the unconverted HF and to neutralize the fluorocarbon compounds before their distillation;
- a column (306) which makes it possible subsequently to extract the F125 (307) at the top, the bottoms (308) subsequently being distilled on the column (309) in order to obtain an F124 + F123 mixture (310) purified from its content of heavy products, the said mixture subsequently being recycled to the reactor (300) in order to be fluorinated therein to F125.

22. Plant according to Claim 21, **characterized in that** it comprises (Fig. 2) a column (207) which makes it possible to purify both the crude F123 (206) resulting from the column (204) of Stage II and the crude F124 + F123 mixture resulting from the bottom of the column (306) of Stage III.
